# EUROPEAN PATENT SPECIFICATION

(11) **EP 4 362 767 B1**
(45) Date of publication and mention of the grant of the patent: **31.12.2025**
(21) Application number: 22751329.8
(22) Date of filing: 13.07.2022
(51) Int. Cl.: A61B 1/005

(54) **DEFLECTION CONTROL MECHANISM FOR A STEERABLE FLEXIBLE ENDOSCOPE, STEERABLE FLEXIBLE ENDOSCOPE, AND METHOD FOR CONTROLLING A FLEXIBLE ENDOSCOPE**
ABLENKUNGSSTEUERUNGSMECHANISMUS FÜR EIN LENKBARES FLEXIBLES ENDOSKOP, LENKBARES FLEXIBLES ENDOSKOP UND VERFAHREN ZUR STEUERUNG EINES FLEXIBLEN ENDOSKOPS
MÉCANISME DE COMMANDE DE DÉVIATION POUR UN ENDOSCOPE FLEXIBLE ORIENTABLE, ENDOSCOPE FLEXIBLE ORIENTABLE, ET PROCÉDÉ DE COMMANDE D'UN ENDOSCOPE FLEXIBLE

(30) Priority: 14.07.2021 DE 102021118193
(43) Date of publication of application: 08.05.2024
(73) Proprietor: Karl Storz SE & Co. KG, 78532 Tuttlingen (DE)
(72) Inventor: TOMBERG, Johan, 78532 Tuttlingen (DE)
(86) International application number: PCT/EP2022/069581
(87) International publication number: WO 2023/285520

(56) References cited:
- WO-A2-2018/022418
- JP-A- H1 147 082
- US-A- 5 507 717
- US-A1- 2007 255 104

## Description

The present invention relates to a deflection control mechanism for a steerable flexible endoscope and to a steerable flexible endoscope having such a deflection control mechanism.

Flexible endoscopes comprise a flexible elongated shaft that is configured for being inserted into an internal cavity of a human or animal body or any other object. In a distal (i.e. distant from a user) end section of the shaft an imaging optics is arranged for generating an image of a scene in the cavity being observed. The endoscopic image generated may be transmitted to a proximal (i.e. close to the user) end of the shaft by a bundle of optical fibers to be picked up by an electronic image sensor positioned in a handpiece arranged at the proximal end of the shaft, or an electronic image sensor may be arranged in the distal end section of the shaft, the image signal being transmitted by electric lines arranged inside the shaft. A flexible endoscope usually comprises an illumination system for illuminating the cavity to be observed, and one or more instrument and/or fluid channels extending from the handpiece through the shaft to its distal end.

Flexible endoscopes are typically employed for observing a cavity being accessed through a curved path, or to observe the interior of an organ that itself has a curved shape such as the intestine, for example. Due to its flexibility the flexible shaft is able to adapt during insertion to the curvature of the access path or the organ. Frequently, however, it is desirable to be able to actively deflect or bend the distal end section of the flexible shaft out of a straight alignment into a bent or articulated shape, in order to facilitate insertion through a curved access path or organ or to control a viewing direction of the imaging optics for observing a desired partial area of the cavity. Endoscopes permitting active deflection or bending of a section of the flexible shaft are usually denoted "steerable endoscopes".

A steerable or deflectable section of the shaft of a steerable endoscope typically has a supporting structure formed by consecutively jointed pivoting elements that can be tipped towards one another by axially movable control wires, or by one or more elastic bending elements which can be bent by the action of control wires. Deflection of the steerable section from an aligned position into a bent or articulated shape in one or an opposite direction can be controlled by reciprocating movement of one pair of counteracting control wires. Frequently, steerable endoscopes permit bending the steerable section in two perpendicular planes by operation of two pairs of control wires. The control wires may extend through the shaft in a proximal direction into the handpiece where a deflection control mechanism is provided. The deflection control mechanism effects movement of the control wires under user control, for example by turning one or more hand wheels.

Frequently it is desirable to maintain a particular articulation angle of the steerable section, for example in order to closely observe or conduct manipulations on a particular area of the cavity. Most preferably such a deflected or bent state of the steerable section can be maintained without the user needing to keep holding the one or more hand wheels, such that the user can operate other control elements or instruments with both hands. The deflected state needs to be maintained against external forces exerted on the shaft by contact with the walls of the cavity, and/or against internal forces due to elasticity of the steerable section itself, for example. When the user releases the one or more hand wheels, the steerable section may undesirably change its deflection angle, due to those external or internal forces. Thus steerable endoscopes have been provided having a deflection control mechanism that comprises a holding or "brake" mechanism to inhibit undesired movement of the control wires, thereby holding the steerable section of the endoscope shaft in a given articulation or to maintain a given deflection angle.

According to EP 3 184026 A1 in an angle adjustment mechanism for an endoscope an outer conical surface which is coaxially aligned with a rotation shaft is provided at an external surface of the rotation shaft, and a conical cylinder, which matches the outer conical surface, is sleeved onto the outer conical surface. A spring, which is capable of driving the rotation shaft to move towards a direction of the conical cylinder and forcing an inner conical surface to closely fit with the outer conical surface, is located along an axial direction of the rotation shaft. An inner end of an endoscope angle adjustment steel wire is fixed to the rotation shaft, and the rotation handle is connected with the rotation shaft.

In JPH 09-98942 an operating device is disclosed for bending an endoscope having a brake mechanism comprising a friction plate being fixed to a friction member in a housing member, whereby frictional force is generated on a face which comes in contact with an inner face of the housing member by pressure from a plate spring. According to JP 2005-160791 a braking mechanism of an endoscope comprises a friction member that can be pressed against a friction surface.

US patent application publication US 2007/0255104 A1 discloses a bent state holding mechanism for an endoscope which includes a control portion and an insertion portion extending therefrom, the insertion portion having a bendable portion at a distal end thereof, including a bendable portion control mechanism provided on the control portion. At least two frictional resistance producing members which rotate relative to each other upon operation of the bendable portion control mechanism are provided, to exert a frictional resistance for holding the bendable portion at any operated position.

JP H 11 47082 discloses an apparatus with the features of the preamble of claim 1.

Known friction-type brake mechanisms, however, frequently face problems of wear, have a complex design, and require time-consuming adjustment or re-adjustment. Moreover, it is often desirable that an articulation angle of the steerable section remains adjustable even when the brake is engaged, wherein dynamic friction should not much exceed static friction, and the user should receive tactile feedback whether the brake is engaged or not.

It is therefore an object of the present invention to provide a deflection control mechanism for a steerable flexible endoscope in which one or more of the above mentioned drawbacks are avoided or alleviated. In particular, it is an object of the invention to provide a deflection control mechanism comprising a brake mechanism having lower wear, being less complex, requiring less adjustment, and/or having improved handling. Further objects of the invention are to provide a steerable flexible endoscope having an improved deflection control mechanism and an improved method for controlling a flexible endoscope.

These objects are met by a deflection control mechanism according to claim 1.

Particular embodiments of the present invention are indicated in the dependent claims.

The present invention relates to medical endoscopes, i.e. to endoscopes designed for medical applications, such as, for example, minimally invasive surgery and/or medical examinations. A user of the endoscope therefore typically is a surgeon conducting an endoscopic intervention. However, the present invention also relates to endoscopes designed for non-medical purposes, for example to industrial endoscopes or borescopes.

According to an aspect of an embodiment of the present invention, a deflection control mechanism is configured for controlling a deflection or articulation or a bending angle of a steerable section of a shaft of a steerable flexible endoscope. The shaft of the endoscope is an elongate, flexible shaft that is configured for being inserted into an internal cavity of a human or animal body or another object such as a technical object, and comprises a steerable section that can be actively bent or deflected with respect to other sections of the shaft. In particular, the steerable section may be actively deflectable with respect to an adjacent section of the flexible shaft under user control. The steerable section may form a distal end section of the shaft, or a section close to the distal end of the shaft, for example. The steerable section may comprise an imaging optics, or an imaging optics may be arranged in an end cap arranged at a distal end of the steerable section, such that by deflecting the steerable section a viewing direction of the imaging optics can be varied. The endoscope may further comprise a handpiece connected to a proximal end section of the shaft, the handpiece having control elements for controlling various functions of the endoscope. An endoscopic image generated by the imaging optics may be transmitted by optical or electronic means to the handpiece. The deflection control mechanism is connected or connectable to the proximal end section of the shaft, and may be accommodated in the handpiece.

The steerable section of the shaft of the flexible endoscope is deflectable by longitudinal movement of at least one elongate control element, which may be a control wire or a pair of counteracting control wires, for example. The steerable section may have a supporting structure of pivoting or elastic elements, for example, which can be angled or bent due to a longitudinal force or a bending moment exerted by the at least one control element. Typically, for controlling deflection in one direction and an opposite direction, two elongate control elements are provided, being embodied as a pair of counteracting control wires that are arranged on opposing sides of the supporting structure, preferably approximately symmetrically to a longitudinal axis of a respective part of the shaft. For controlling the deflection or bending of the steerable section the control wires are movable relative to the shaft substantially in their respective longitudinal direction, which direction usually is parallel to the axial direction of the respective part of the shaft. Thus, when a first one of the two wires of the pair is pulled in the proximal direction for deflection to one side, the other one will move in the distal direction; for deflection to the opposite side, the other one of the two wires is pulled in the proximal direction, while the first one will move in the distal direction. The at least one control element, for example the control wires, may be guided in the shaft to the proximal end of the shaft, being operable in opposing or counteracting manner by the deflection control mechanism.

The deflection control mechanism comprises a support structure, a drive wheel, and a hand wheel. The support structure may be, for example, a base structure of a handpiece, or may be connected to the base structure or to a housing of the handpiece. The drive wheel is rotatably supported in or on the support structure, for example with one or more bearings, having a rotatable shaft and/or an axle fixed to the support structure. The deflection control mechanism is connected or is connectable to the at least one elongate control element to effect movement of the at least one elongate control element by rotation of the drive wheel. Thus, for example, the at least one control element may be would around the drive wheel, or an elongate traction element may be wound around the drive wheel having at least one coupling section for coupling to a proximal end of the at least one control element, such that by rotation of the drive wheel the at least one control element can be moved in its longitudinal direction for controlling deflection of the steerable section. A pair of counteracting control wires may be formed by respective sections of a continuous control wire, the control wire being wound around the drive wheel, or the traction element may comprise respective coupling sections at its both ends for coupling to a respective one of a pair of counteracting control wires. A rotation axis of the drive wheel preferably is transverse to a longitudinal direction of the at least one control element. Regarding the aforementioned aspects the deflection control mechanism may be embodied, for example, as described in German patent application DE 10 2020 118 043.8.

The hand wheel is rotationally coupled to the drive wheel, such that the drive wheel can be rotated by rotating the hand wheel. Thus, by manually turning the hand wheel the deflection angle of the steerable section of the shaft is controllable. Preferably the hand wheel is arranged co-axial with the drive wheel, being supported in or on the support structure. For ease of operation the hand wheel may comprise protrusions, recesses and/or grooves at its outer circumference.

The deflection control mechanism further comprises a brake stator element rotationally coupled to the support structure and a brake rotor element rotationally coupled to the drive wheel and, consequently, rotationally coupled to the hand wheel. The brake rotor element may be rigidly connected to the hand wheel. With respect to the support structure, therefore, the brake rotor element is rotatable and can be rotated by turning the hand wheel, while the brake stator element is non-rotatable. As employed here, the term "rotationally coupled" means, in particular, that an element is connected to another one such that one of the elements can be rotated by rotating the other one or that both are non-rotatable, and the term "non-rotatable" may imply that the respective elements cannot be rotated with respect to one another, except for a possible play of a mechanical connection of both elements. Preferably the brake rotor element is arranged co-axial with the drive wheel, being supported in or on the support structure; most preferably, the hand wheel, the brake rotor element, and the drive wheel have a common axis of rotation. The brake rotor element may be rigidly connected to the drive wheel via a rotatable shaft.

The brake stator and brake rotor elements are displaceable relative to one another in an axial direction. Thus, for example, the brake rotor element may be rotatable, but axially fixed with respect to the support structure, and the brake stator element may be non-rotatable but displaceable in the axial direction with respect to the support structure. On the other hand, the brake rotor element may be rotatable and axially movable, and the brake stator element may be rotationally and axially fixed with respect to the support structure. Here and in the following the terms "axial" and "radial" refer to the rotation axis of the brake rotor element.

A first one of the brake stator and brake rotor elements comprises an elastically deformable lip, and the other one of the brake stator and brake rotor elements comprises a friction surface, wherein the lip is arranged for being pressed against the friction surface by axial displacement of the brake stator element relative to the brake rotor element, thereby effecting, in particular, frictional rotational coupling of the brake rotor element to the brake stator element. Thus, the deflection control mechanism is configured such that the brake stator element can be axially displaced relative to the brake rotor element such that the lip is pressed against the friction surface to effect frictional engagement of the brake stator and brake rotor elements with one another. In particular, relative to the support structure, the brake stator element may be displaceable towards the brake rotor element, and/or the brake rotor element may be displaceable towards the brake stator element, to provide the frictional engagement. The elastically deformable lip may be arranged adjacent to and facing the friction surface, being configured to be pressed against the friction surface in a ring-shaped area of the friction surface by axial movement of the brake stator element towards the brake rotor element, for example. By axial movement in an opposite direction the lip may be removable from the friction surface. Preferably, frictional coupling of the brake rotor element with the brake stator element is effected only by the lip contacting the friction surface.

Thus, in a first state of the deflection control mechanism the lip may be detached from the friction surface or contacts the friction surface only lightly, i.e. under substantially no pressing force. In a second state, the lip is pressed against the friction surface under an axial pressing force, providing the frictional coupling with the friction surface. The deflection control mechanism is configured such that the second state can be reached by displacing the brake stator element relative to the brake rotor element in the axial direction; moreover, the deflection control mechanism may be configured for generating an axial force for pressing the lip against the friction surface. In particular, the brake stator element may be displaceable in the axial direction relative to the brake rotor element under an axial force that provides an axial pressing force. When the lip is pressed to the friction surface under the action of the axial pressing force, it is elastically deformed and thereby generates an elastic force counteracting the axial displacement of the brake stator element relative to the brake rotor element. When the axial force exerted on the brake stator and/or rotor element is reduced, the elastic force acts to displace the brake stator element relative to the brake rotor element in an opposite axial direction, i.e. the lip acts as a spring to push both brake elements away from one another. When the axial force is completely released, the lip may be substantially detached from the friction surface, the deflection control mechanism returning to its first state.

The deflection control mechanism thus comprises a brake mechanism, the brake mechanism comprising the brake stator element and the brake rotor element, one of which comprises the elastically deformable lip and the other one having the friction surface. In the following the first state, when the lip is separate from the friction surface or contacts it only lightly, is referred to as the "brake-off" state of the brake mechanism, while the second state, when the lip is pressed against the friction surface, is referred to as the "brake-on" state. The brake mechanism may include further elements serving, for example, for effecting the axial displacement and/or for generating the axial pressing force.

An amount or degree of frictional coupling, i.e. a maximal torque that can be transmitted by the frictional coupling between the brake stator element and the brake rotor element, depends on the axial pressing force and therefore depends on an amount of the axial displacement, i.e. on an axial position, of the brake stator element relative to the brake rotor element. Rotation of the brake rotor element relative to the support structure is blocked up to the maximal torque transmitted by the frictional coupling, whereas slippage occurs when a higher torque is exerted on the brake rotor element. In general, the maximal torque can be increased by reducing an axial separation between the brake stator and brake rotor elements, thereby pressing the lip with an increased axial force against the friction surface. Conversely, the amount of frictional coupling generally can be reduced and thus the maximal torque decreased by increasing an axial separation between the brake stator and brake rotor elements, thereby reducing the axial pressing force. Material and/or geometrical properties of the lip, as well as a total range of axial displacement of the brake stator element relative to the brake rotor element can be chosen to achieve a desired braking torque and slippage characteristics.

Due to one of the brake stator and rotor elements comprising an elastically deformable lip, being configured and arranged for being pressed against a friction surface of the other one of both brake elements by axial movement of the brake stator element relative to the brake rotor element, a braking torque can be generated for inhibiting rotation of the drive wheel and the hand wheel relative to the support structure. Consequently, movement of the at least one control element is inhibited, and the steerable section of the endoscope shaft is held at a given articulation angle, relieving the user from holding the hand wheel to maintain the deflection. Thus the user is free to employ both hands for operating other control elements or instruments.

As the brake mechanism works by frictional coupling, the steerable section can be held fixed but nevertheless remains adjustable when the brake is on. In particular, the brake can be overcome by turning the hand wheel with such a torque that the maximal torque is exceeded that can be transmitted by the frictional coupling of the brake rotor element to the brake stator element. The user therefore may be able to operate the deflection control mechanism by simply turning the hand wheel at a sufficient torque, without needing to take any action to unlock the brake. Similarly, by exerting a sufficiently high external force on the steerable section of the endoscope shaft, a torque on the drive wheel can be generated such that the maximal torque of the frictional coupling is exceeded, and the steerable section can be bent or straightened without releasing the brake. The braking torque may be chosen such that it is higher than a corresponding torque required for holding the steerable section at a given deflection angle, but less than a torque than can conveniently be exerted by the user by turning the hand wheel and, possibly, small enough such that the steerable section can adapt itself to a shape of a cavity or an access path even if the endoscope is withdrawn without releasing the brake.

By relative displacement of the brake stator and brake rotor elements in an axial direction to withdraw both brake elements from one another, the frictional engagement of the lip with the friction surface can be removed. When an axial force pressing the lip against the friction surface is released, the brake stator and rotor elements are pushed away from one another by the elastic force generated by the lip acting as a spring. Frictional coupling is thus ceased or at least diminished; a small amount of friction may remain due to the lip contacting the friction surface even without an axial force. The brake can thus be released by correspondingly moving the brake stator element relative to the brake rotor element, such that the user can resume controlling the steerable section in the usual way. An amount of resistance the user feels when turning the hand wheel provides haptic feedback about whether the brake is on or off.

The deflection control mechanism in accordance with the present invention permits simple, intuitive, and safe operation of the flexible endoscope. Locking and unlocking the brake may be accomplished in a particularly simple and safe manner, wherein the elastic resilience of the lip may be sufficient to release the frictional engagement when the brake is unlocked, such that no dedicated spring element is required. Moreover, the deflection control mechanism may have a comparatively simple design permitting simple and cost-efficient manufacturing. In particular, manufacturing tolerances can be compensated for at least to some degree by the elastically deformable lip, thus eliminating or reducing the need for adjustment. The lip may be injection-molded, and elements of the brake mechanism may be made of sheet metal, thus further reducing manufacturing cost.

The deflection control mechanism may be configured for effecting movement of more than one pair of counteracting control wires. A shaft coupling may be provided at a distal side of the handpiece or the deflection control mechanism for coupling the endoscope shaft to the handpiece and/or the deflection control mechanism. The deflection control mechanism may be hermetically sealed by suitable seals, for example by seal rings inserted between rotatable elements, to increase durability.

In accordance with an advantageous embodiment of the invention, the brake stator element comprises the elastically deformable lip, and the brake rotor element comprises the friction surface. In particular, the brake stator element may be axially displaceable with respect to the support structure, and the brake rotor element may be fixed in the axial direction relative to the support structure. The brake stator element may be guided in or on the support structure to be movable in the axial direction. Thus the elastically deformable lip is axially movable, but non-rotatable relative to the support structure, while the friction surface is rotatable, but axially fixed.

In the following some aspects of embodiments of the invention are described referring to the aforementioned embodiment, the lip being axially movable, but non-rotatable with respect to the support structure, while the friction surface is rotatable and axially fixed. It is to be understood, however, that the invention relates as well to such embodiments with the lip being comprised by the brake rotor element and the brake stator element having the friction surface; further, the invention also relates to embodiments with the brake stator element being axially fixed and the brake rotor element being axially displaceable with respect to the support structure, or both of the brake stator and brake rotor elements being axially displaceable.

Preferably the friction surface is a substantially planar radial surface, i.e. the friction surface preferably extends at least approximately in a plane perpendicular to the axial direction, forming at least approximately a planar surface. In particular, the brake rotor element may be substantially disk-shaped, having the friction surface on that side that faces the lip, such that the lip can contact and can be pressed against the friction surface by axial movement. The surface of the brake rotor element preferably is a substantially planar radial surface at least in a ring-shaped section to be contacted by the lip. The brake rotor element may be formed by a substantially disk-shaped plate that carries the hand wheel, the disk-shaped plate being rigidly connected to the hand wheel and being rotatable with respect to the support structure. The disk-shaped plate forming the friction surface may be made of metal, for example of stainless steel. This embodiment has a particularly simple, compact, and robust design.

According to an advantageous embodiment, the first one of the brake stator and brake rotor elements comprises a brake pad holder to which a brake pad is attached, wherein the brake pad comprises the elastically deformable lip. In particular, the brake pad may be embodied as the elastically deformable lip or may carry the lip; the lip may be an axial projection or elevation of the brake pad projecting towards the friction surface. The brake pad holder may have a disk portion and a cylindrical portion, wherein the brake pad is fixed to the disk portion and/or is at least partially encompassed by the cylindrical portion, thereby firmly holding the brake pad in the brake pad holder. The lip preferably protrudes in the axial direction over the cylindrical portion. Advantageously, the brake pad holder is configured and arranged symmetrically with respect to the axis of rotation of the brake rotor element. The brake pad holder may be substantially rigid and may be made of metal, for example stainless steel. In particular, the brake stator element may comprise the pad holder, the brake pad holder being rotationally fixed, but axially movable with respect to the support structure. In this way handling and durability of the brake mechanism can be further enhanced.

The brake pad, according to an exemplary embodiment, comprises a base portion that carries the elastically deformable lip, such that the lip at its free side forms a contact edge or contact rim for contacting the friction surface. The lip may be connected to the base portion of the brake pad at a base side of the lip, and at its opposite side, the lip has the contact edge or contact rim protruding in the axial direction towards the friction surface. Thus, when the brake stator element approaches the friction surface, the contact edge contacts the friction surface first; i.e., in the absence of an axial force pressing the lip against the friction surface or in the brake-off state, the lip contacts the friction surface at most at the contact edge or contact rim, while other portions of the lip remain separated from the friction surface. The base portion preferably is substantially ring-shaped being arranged symmetrically with respect to the axis of rotation of the brake rotor element. Most preferably the contact edge or rim is arranged near an outer circumference of the brake pad. In this way smooth operation of the brake mechanism may be achieved, the deflection control mechanism having low friction when the brake is off.

The elastically deformable lip preferably is substantially ring-shaped, being arranged symmetrically with respect to the axis of rotation of the brake rotor element; most preferably the lip forms a circular ring extending over a full circle having constant radius. In this way a braking force can be maximized. Alternatively, the lip may extend over one or more sections of a full circle only and/or have interruptions or gaps.

The elastically deformable lip has an original or undeformed state it has in the absence of an axial pressing force, which in the following is denoted the uncompressed state, and a deformed state that it has when being pressed against the friction surface, which in the following is denoted the compressed state. In the uncompressed state the lip has its original shape, while in the compressed state it assumes a shape that may depend, i.a., on the axial force pressing the lip against the friction surface and on the shape of the friction surface. Moreover, the lip may have a multiplicity of intermediate or semi-compressed states corresponding to intermediate axial pressing forces. On its side facing the friction surface the lip has a contact surface for contacting the friction surface when approaching the friction surface by axial displacement of the brake stator element relative to the brake rotor element, such that at least a section of the contact surface contacts the friction surface in the compressed state of the lip.

The contact surface of the elastically deformable lip in its uncompressed state has, at least approximately, a frustoconical shape. The truncated cone formed by the substantially frustoconical contact surface preferably is arranged at least approximately co-axial with the axis of rotation of the brake rotor element. Thus, in particular, before axial movement relative to the brake rotor element or when no axial force is exerted on the brake stator element, i.e. when the brake is off, the lip contact the friction surface at most at its contact edge or rim; when the brake stator element is fully displaced towards the brake rotor element, i.e. when the brake is on, the lip is deformed under the action of an axial force pressing the lip against the friction surface, such that at least a section of the contact surface of the lip contacts the friction surface. At an intermediate displacement or axial force, a smaller section of the contact surface may contact the friction surface. A braking torque generated by the frictional coupling may gradually increase when the brake stator element is moved towards the brake rotor element, and thus, for example, with decreasing separation between the brake pad holder and the disk having the friction surface. Due to its elasticity and acting as a spring, the elastic lip in its compressed state generates a counteracting force that, when the pressing force is released, displaces the brake stator element in the opposite axial direction, thereby reducing the contacting section and the frictional coupling. The braking torque therefore gradually decreases with increasing separation, i.e. by displacement of the brake stator element relative to the brake rotor element in the opposite axial direction. In this way handling of the brake mechanism can be further enhanced.

Preferably the frustoconical contact surface is arranged such that an outer circumference of the frustoconical surface is closest to the friction surface. In particular, an outer circumference of the frustoconical surface may form the contact edge or contact rim of the lip that contacts the friction surface first when the lip approaches the friction surface by axial displacement of the brake stator element relative to the brake rotor element. An opening angle of the truncated cone may be adapted to a deformability of the lip and may be chosen, for example, to provide smooth increase and decrease of the braking torque, as well as a suitable maximal torque for holding the steerable section of the endoscope shaft at a given articulation angle when the lip is fully compressed, i.e. when a maximal range of movement of the brake stator element and/or the brake rotor element has been reached. In this way the brake mechanism can be adapted to particular needs.

The elastically deformable lip preferably comprises a cone portion having the frustoconical contact surface on a side facing the friction surface, or the lip is embodied as the cone portion. The cone portion is deformable by pressing the lip against the friction surface, such that the frustoconical surface may be elastically flattened and eventually adapted to the shape of the friction surface. In case of the friction surface being a radial plane surface, at least a section of the contact surface may attain a planar shape, forming a substantially planar contact area. In this case the cone section may be deformed at least partially into a planar shape extending in a radial direction, i.e. in the compressed state of the lip the contact surface may form or comprise a substantially planar radial surface. The lip may comprise a stem portion connected to the base portion of the brake pad, the cone portion being connected to the stem portion. The cone portion may have uniform thickness. The stem portion and the cone portion may be formed in one piece, and the lip and the base portion of the brake pad may be formed in one piece. In particular, a diameter of the cone portion may be chosen to generate a desired braking torque when the lip is pressed against the friction surface. In this way the brake mechanism is further adaptable to particular applications.

Advantageously the elastically deformable lip may comprise an elastomeric material. Alternatively or additionally, the lip may comprise a composite material, such as, for example, an elastomeric material reinforced by a steel wire, which may be made of spring steel to generate the elastic force or to modify a spring rate of the lip. In particular, the lip may consist of such an elastomeric or composite material. The lip may be injection-molded, preferably being formed in one piece, wherein in a composite material the steel wire may be molded in, permitting cost-efficient manufacturing. Most preferably, the brake pad is injection-molded, being made in one piece including the lip. The elastomeric or composite material may be chosen to be resistant to the mechanical, chemical and thermal conditions of medical sterilization processes, and to provide specific elastic and frictional properties as required for a specific application. Suitable materials may be, for example, PUR (polyurethane), some form of TPE (thermoplastic elastomers), FKM or similar fluoroelastomers, EPDM (ethylene propylene diene monomer rubber), or silicone. The elastomeric and/or composite material may be provided with a color code depending on the specific material, thus facilitating manufacturing a series of endoscopes with application-specific brake properties.

The materials and surface structures of the lip and the friction surface may be chosen to provide low wear and suitable frictional properties. Preferably, the friction surface exhibits a coating for controlling friction with the contact surface of the lip. In particular, lubrication may be provided by a lubricant dissolved in a solvent used to clean the friction surface during assembly of the deflection control mechanism, leaving a coating on the friction surface after evaporation of the solvent. Mineral solvent such as white spirit has also been found to provide favorable lubrication, leaving residue on the friction surface that affects the frictional properties. **In** this way static and dynamic friction can be leveled out, providing smooth operation of the deflection control mechanism.

According to an advantageous embodiment, the deflection control mechanism comprises a brake toggle plate co-operating with the brake stator element to effect axial displacement of the brake stator element by rotation of the toggle plate with respect to the support structure. Alternatively, the brake toggle plate may co-operate with the brake rotor element to displace the brake rotor element in an axial direction. The brake toggle plate, as well as the toggle operation element, preferably is rotatable with respect to the rotation axis of the brake rotor element. The brake toggle plate may be operationally coupled to a toggle operation element or a brake switch such as a lever or knob that can be manually operated by the user for switching the brake on or off. In particular, the brake stator element may have a wedge structure such as, for example, at least one sloped notch or groove, and the brake toggle plate may have at least one cam element such as, for example, a nub. The cam element engages the wedge structure, being pushed out of the wedge structure by rotation of the toggle plate with respect to the brake stator element to axially displace the brake stator element, thereby translating rotation of the toggle plate into linear axial displacement of the brake stator element. Conversely, the brake toggle plate may have the wedge structure and the brake stator element may exhibit the at least one cam element, effecting axial displacement of the brake stator element in a similar manner. Most preferably, the wedge structure comprises at least one detent structure, for example a recess or indentation, such that the cam element in a defined rotational position of the toggle plate engages with the detent structure, thereby haptically marking the respective rotational position. The at least one detent structure may correspond to the brake-on and/or the brake-off state, marking brake-on and/or brake-off rotational positions of the brake toggle plate. Alternatively or additionally, a stop may be provided limiting a range of rotation of the brake toggle plate and thus limiting a range of axial displacement of the brake stator element relative to the brake rotor element. Thus simple and reliable switching of the brake mechanism including haptic feedback can be facilitated.

Further advantageously, the brake toggle plate may have at least one pre-determined intermediate position. The intermediate rotational position of the brake toggle plate corresponds to an intermediate axial position of the brake stator element relative to the brake rotor element in which the lip is pressed at an intermediate axial force against the friction surface, providing an intermediate braking torque that is less than a maximal braking torque provided when the lip pressed at a maximal axial force to the friction surface. Thus, in accordance with an exemplary embodiment, the brake toggle plate has a brake-off rotational position relative to the brake stator element in which the lip is detached from the friction surface or at least subjected to substantially no axial force, a brake-on position in which the lip is pressed at a maximal axial force against the friction surface, and an intermediate position in which the lip is pressed at an intermediate force against the friction surface. The pre-determined intermediate rotational position of the brake toggle plate may be defined by a corresponding detent structure of the wedge structure, such that the cam element engages with the detent structure in the intermediate rotational position, thereby haptically marking the intermediate rotational position. In particular, the wedge structure may comprise a first detent structure marking the brake-off position of the brake toggle plate, a second detent structure marking the brake-on position, and at least one intermediate detent structure marking the at least one intermediate rotational position. Preferably the brake toggle plate can be manually rotated by a toggle operation element such as a lever or knob, the toggle operation element having corresponding brake-on, brake-off and intermediate rotational positions. In case that the brake toggle plate co-operates with the brake rotor element, the brake-on, brake-off and intermediate rotational positions of the brake toggle plate refer to the brake rotor element. The pre-determined intermediate position corresponds to the brake being semi-engaged, i.e. providing a pre-determined intermediate braking torque, thereby further improving handling of the brake mechanism.

In accordance with a preferred embodiment of the invention, the deflection control mechanism comprises a further drive wheel rotatably supported in or on the support structure, preferably co-axially with the aforementioned drive wheel, and a further hand wheel that is rotationally coupled to the further drive wheel and that also may be arranged co-axially with the aforementioned hand wheel. Moreover, according to this embodiment, the deflection control mechanism comprises a further brake stator element rotationally coupled to the support structure and a further brake rotor element rotationally coupled to the further drive wheel. The further brake stator and brake rotor elements are displaceable relative to one another in an axial direction, referring to a rotation axis of the further brake rotor element that preferably coincides with the rotation axis of the aforementioned brake rotor element. Further in accordance with this embodiment, a first one of the further brake stator and brake rotor elements comprises a further elastically deformable lip and the other one of the further brake stator and brake rotor elements comprises a further friction surface. The further elastically deformable lip is arranged for being pressed against the further friction surface by axial displacement of the further brake stator element relative to the further brake rotor element thereby effecting, in particular, frictional coupling of the further brake stator and brake rotor elements to generate a braking torque. The further drive wheel, the further hand wheel, the further brake stator and brake rotor elements, as well as the further elastically deformable lip and the further friction surface, may be configured like the respectively corresponding elements described above. The deflection control mechanism according to the present embodiment therefore may be considered to comprise, in addition to the elements of the deflection control mechanism according to any one of the above described embodiments, further elements constituting a further deflection control mechanism, having a common support structure, the further deflection control mechanism being equipped with a further brake mechanism. The deflection control mechanism may be configured for controlling deflection of the steerable section of an endoscope shaft that comprises at least two elongate control elements for controlling deflection of the steerable section in two different planes, wherein at least a first elongate control element can be operated by rotating the hand wheel as described above for controlling deflection in a first plane and at least a second elongate control element can be operated by rotating the further hand wheel for controlling deflection in a second plane. The steerable section can be held at a given deflection angle in the first and/or second plane by the brake mechanisms relating to the aforementioned hand wheel and/or to the further hand wheel, respectively. The first and second planes may be at an angle of 90° to each other. Thus, the steerable section can be deflected and held in any arbitrary direction.

The deflection control mechanism may comprise further features such as a stop mechanism as disclosed in German patent application DE 10 2020 118 047.0, and/or a modular plate structure as disclosed in German patent application DE 10 2020 118 048.9, which are hereby incorporated by reference into the present application.

According to a further aspect of the present invention, a steerable flexible endoscope is provided that has an elongate flexible shaft comprising a steerable section that is deflectable or bendable by longitudinal movement of at least one elongate control element, wherein the flexible endoscope further comprises a deflection control mechanism configured as described above. In particular, the endoscope may comprise a handpiece connected to a proximal end of the flexible shaft, the handpiece accommodating the deflection control mechanism, wherein the proximal end of the at least one elongate control element is operatively connected to the deflection control mechanism. Most preferably, the steerable section is deflectable in two perpendicular planes by movement of at least two elongate control elements, and the deflection control mechanism is configured for operating the at least two elongate control elements for controlling deflection in the two perpendicular planes as described before.

The present invention further relates to a method for controlling a flexible endoscope, the flexible endoscope having an elongate flexible shaft comprising a steerable section that is deflectable by movement of at least one elongate control element, for example by longitudinal movement of at least one pair of control wires, and wherein the flexible endoscope comprises a deflection control mechanism for controlling movement of the at least one elongate control element, the deflection control mechanism being configured as described above. In accordance with the method, the steerable section may be deflected to a desired articulation angle by turning the hand wheel. In order to hold the steerable section at the given articulation angle, the brake stator element is displaced in the axial direction relative to the brake rotor element, for example by rotating a brake toggle plate configured as described above, such that the elastically deformable lip is pressed against the friction surface. Thereby the brake stator and brake rotor elements are frictionally coupled to each other, generating a braking torque to hold the steerable section. In this situation it may nevertheless be possible to deflect or straighten the steerable section by applying a torque on the hand wheel that is sufficient to overcome the braking torque. Further in accordance with the method, the brake stator element may be displaced in the opposite axial direction relative to the brake rotor element, for example by rotating the brake toggle plate in an opposite direction, such that the elastically deformable lip is released from the friction surface. Thereafter the steerable section can be further deflected or straightened by again turning the hand wheel in a corresponding direction. The method provides particularly safe and convenient operation of the flexible endoscope.
Fig. 1 shows a steerable flexible endoscope in an overall view;
Fig. 2 shows a deflection control mechanism in accordance with an exemplary embodiment of the present invention;
Fig. 3 shows a proximal part of the deflection control mechanism of Fig. 2 in an enlarged sectional view;
Fig. 4a and 4b show a sectional view of the brake mechanism relating to the first hand wheel of the deflection control mechanism of Fig. 3 in the brake-off and the brake-on states, respectively;
Figs. 5a and 5b show the brake pad of the brake mechanism shown in Figs. 4a and 4b in a sectional view in the brake-off and the brake-on states, respectively;
Figs. 6a and 6b show the brake toggle mechanism of the first hand wheel shown in Figs. 4a and 4b in a partially transparent perspective view in the "brake-off" and the "brake-on" states, respectively.

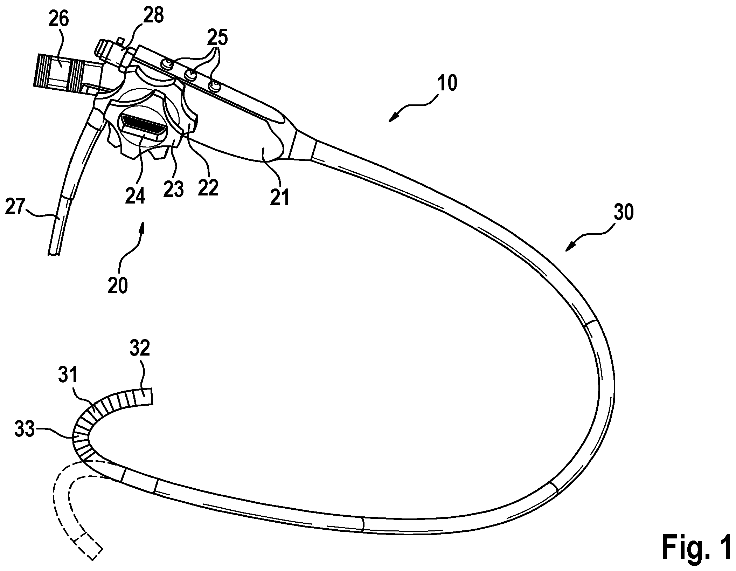

As shown schematically in Fig. 1, a flexible endoscope 10 typically comprises a handpiece 20 and an elongate flexible shaft 30, the handpiece 20 being attached to a proximal end of the shaft 30. The handpiece 20 has an outer housing 21 made of a plastic and/or metallic material. On a lower side of the housing 21 a first hand wheel 22 and a second hand wheel 23 are arranged for controlling a deflection of a steerable section 31 of the shaft 30, as is described below. Typically the first and the second hand wheel 22, 23 are arranged co-axially, and at an exterior side of the second hand wheel 23 a knob 24 for controlling a deflection brake relating to the second hand wheel 23 is provided. Between the housing 21 and the first hand wheel 22 a brake lever 42 is arranged for operating a deflection brake relating to the first hand wheel 22 (see below, not shown in Fig. 1). Both hand wheels 22, 23, the brake knob 24, and the brake lever 42 can be operated with one hand. Further, the handpiece 20 may exhibit a multiplicity of control buttons 25 for controlling various functions of the flexible endoscope 10, such as for controlling the imaging and/or illumination system and/or irrigation and suction pumps, for example. The handpiece 20 may be connectable to an external video unit or a video monitor via connector 26 and to an external light source via light cable 27. Moreover, an instrument port 28 may be provided for inserting endoscopic instruments to be advanced through one or more respective channels to a distal end of the shaft 30 for manipulating tissue or other objects within a cavity into which the shaft 30 can be inserted.

At its distal end the flexible shaft 30 comprises a steerable section 31. The steerable section 31 may form a distal end section of the shaft 30 or, as shown in Fig. 1, may carry a distal end cap 32 which may accommodate an imaging optics and an electronic image sensor for providing an endoscopic image of a cavity into which the shaft 30 is inserted. The image signal generated by the image sensor may be transmitted via electric cables extending through the shaft 30 and the handpiece 20 to the connector 26 for being processed and displayed by an external video unit. The shaft 30 in total is flexible to an extent to be advanced through an endoscopic access or a hollow organ towards a cavity to be observed, being capable of adapting to a curved shape of the access or the organ. The steerable section 31, on the other hand, can be flexed actively by turning the hand wheels 22, 23. To this end the steerable section 31 comprises an inner structure of a multiplicity of consecutive pivoting elements 33, thus being deflectable in one or more planes. The pivoting elements 33 are covered by a flexible tube to form a smooth outer surface.

For controlling the deflection of the steerable section 31 two counteracting control wires (not shown in Fig. 1) are provided extending on opposite sides within the steerable section 31, by a longitudinal movement of which the steerable section 31 can be bent to one or the other side, as indicated symbolically in Fig. 1. The control wires extend along the shaft 30 and are connected at their proximal ends to a deflection control mechanism arranged within the handpiece 20 which can be operated by the user by turning the hand wheels 22, 23 for deflecting the steerable section 31. In the exemplary embodiment shown, the steerable section can be bent within a first plane that corresponds to the plane of the drawing, a deflection angle being controllable by rotating the first hand wheel 22. Further, the steerable section 31 can be bent in a second plane perpendicular to the first plane and perpendicular to the plane of the drawing, a corresponding deflection angle being controllable by rotating the second hand wheel 23.

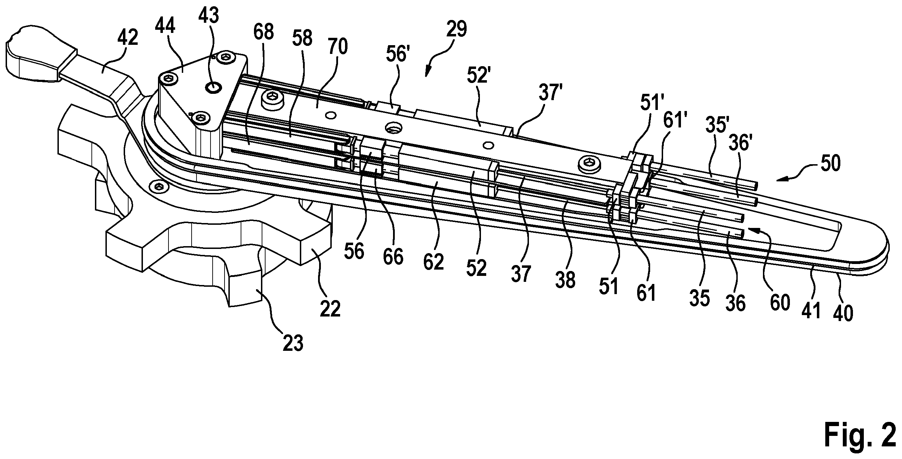

Fig. 2 shows a handpiece 20 of a flexible endoscope in accordance with an exemplary embodiment of the invention, wherein an upper part of the housing 21 of the handpiece 20 and an inner half-shell housing have been removed to show elements of the deflection control mechanism 29. The handpiece 20 may comprise further elements not shown in Fig. 2 (see Fig. 1). As depicted in Fig. 2, a lower part of the housing is formed by a handpiece body 40 preferably formed of metal such as stainless steel, for example. At its lateral sides the body 40 carries a seal ring 41 of an elastic seal material to form a sealed connection with the upper part of the housing; thus, an interior space of the handpiece 20 accommodating the deflection control mechanism 29 is sealingly enclosed. At a lower side of the body 40 the first hand wheel 22 and the second hand wheel 23 are rotatably mounted, having a common axis of rotation. The common axis is defined by an axle 43 that is non-rotating and fixedly held in a rigid metal cage 44. The axle 43 defines a common rotation axis of two drive wheels (sprockets 69, 59) that can be rotated by turning the first and second hand wheels 22, 23, respectively, as described below in detail.

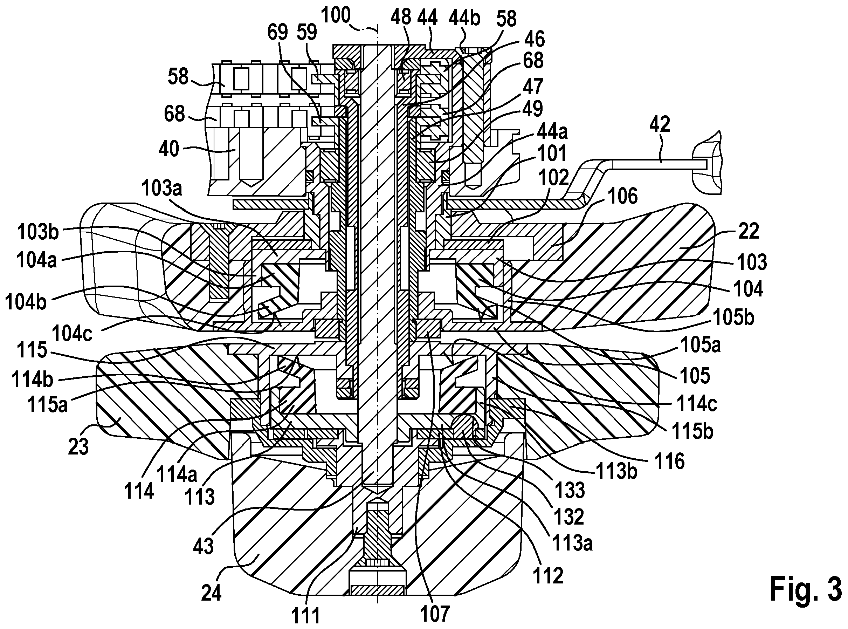

Moreover, in Fig. 2 the proximal end sections of the sheaths 35, 35', 36, 36' of two pairs of counteracting control wires are shown; the control wires continue to the right-hand side of the drawing and extend through the shaft 30 of the endoscope to the steerable section 31 (see Fig. 1). The wire sheaths 35, 35', 36, 36' are each firmly held in a respective wire coil mounting 51, 51', 61, 61'. The proximal end sections of the control wires 37, 38 protrude from the respective sheaths 35, 36. In corresponding manner the proximal end sections of respective counteracting control wires of the two pairs of control wires protrude from the respective sheaths 35', 36', only control wire 37' being visible in Fig. 2. The control wires are each fixed in a respective fixation element (rectangular fixation blocks 52, 52') connected to a chain 58. The chain 58 engages with the teeth of a drive wheel (sprocket 59, see Fig. 3) and is wound around the drive wheel, enclosing an angle of about 180°, thus connecting the fixation block 52 to the corresponding fixation block 52' on the other side of the deflection control mechanism. The chain 58 consists of a multiplicity of segments connected to each other by hinges, which are not shown in Fig. 2 for simplicity. Further, a stopping block 56 may be provided connected to the fixation block 52 and, likewise, a stopping block 56' connected to the fixation block 52', the stopping blocks 56, 56' co-operating with corresponding fixed stops (not shown). The fixation blocks 52, 52' and stopping blocks 56, 56' each have substantially rectangular cross section and may be guided in correspondingly shaped channels. Further, a tensioning mechanism may be provided, such that the control wires 37, 37' are subjected to a pre-tension.

By rotation of the sprocket 59, both ends of the chain 58 and connected coupling sections formed by elements 52 and 56 and corresponding elements 52' and 56', respectively, are moved in opposing directions, thus effecting corresponding opposing movement of the control wires 37, 37'. In this way deflection of the steerable section 31 of the endoscope 10 in a first plane can be controlled (see Fig. 1).

In the present example the deflection control mechanism 29 comprises two layers 50, 60, an upper layer 50 being configured for controlling the deflection of the steerable section 31 in the first plane by reciprocating longitudinal movement of the upper pair of control wires 37, 37', and a lower layer 60 being configured for controlling a deflection of the steerable section 31 in a second plane, perpendicular to the first plane, by reciprocating movement of the lower pair of control wires (of which only control wire 38 is shown in Fig. 2). The lower layer 60 is configured in a corresponding manner as described above (only fixation block 62, stopping block 66, and chain 68 being visible in Fig. 2), such that deflection of the steerable section 31 of the endoscope 10 in a direction perpendicular to the first plane can be controlled by movement of the lower pair of control wires 38. Both layers 50, 60 comprise elements of a support structure including one or more plates 70 mounted on an upper side of the body 40. The upper layer 50 of the deflection control mechanism 29 is operated by turning the second hand wheel 23, while the lower layer 60 of the deflection control mechanism 29 is operated by turning the first hand wheel 22.

The deflection control mechanism 29 comprises a brake mechanism that is shown in Fig. 3 in an enlarged sectional view drawn in a vertical plane that includes a rotational axis 100 of the sprockets 59, 69 and extends in a longitudinal direction of the handpiece 20, i.e. of the deflection control mechanism 29 (see Figs. 1 and 2). The brake mechanism comprises a brake mechanism relating to the lower layer 60 and a further brake mechanism relating to the upper layer 50. The rotational axis of the sprockets 59, 69 also is the rotational axis of respective articulation shafts 46, 47, as is described below. In the following description the terms "axial" and "radial" refer to the common rotational axis 100 of the sprockets 59, 69 and articulation shafts 46, 47. In the exemplary embodiment shown, hand wheels 22, 23, lever 42 and knob 24 are rotatable with respect to the axis 100.

As can be seen in Fig. 3, the sprocket 59 of the upper layer 50 is rigidly held on an articulation shaft 46 extending to a lower side of the handpiece 20. The articulation shaft 46 is connected to the second hand wheel 23, for being rotated by turning the second hand wheel 23. Likewise a sprocket 69 forming a drive wheel of the lower layer 60 of the deflection control mechanism is fixed on a further articulation shaft 47 extending coaxially with the articulation shaft 46 into the first hand wheel 22 with which it is connected for being rotated by turning the first hand wheel 22. The chains 58, 68 are engaged with the teeth of the sprockets 59, 69, respectively. The articulation shaft 46 and thus the sprocket 59 of the upper layer 50 of the deflection control mechanism 29 are supported by a ball bearing 48 on the rigid axle 43 fixedly held in the cage 44. The articulation shaft 47 of the lower layer 60 is held by a ball bearing 49 in a cage body 44a that is firmly connected to the cage 44. The cage 44 and the cage body 44a are firmly held to the handpiece body 40 by a screw 44b, forming a rigid support structure that includes the cage 44, cage body 44a, the axle 43, the handpiece body 40, and the one or more plates 70.

The brake mechanism relating to first hand wheel 22, i.e. to the lower layer 60 and the lower pair of control wires 38, comprises a brake lever 42 fixedly mounted on a brake lever shaft 101 which is pivotably held on a lower section of the cage body 44a. An approximately circular plate, which in the following is denoted brake toggle plate 102, is rigidly connected to the brake lever shaft 101, thus being rotationally coupled to the brake lever 42. Adjacent and parallel to the brake toggle plate 102 a brake pad holder 103 is mounted on the lower section of the cage body 44a in a rotationally fixed, but axially movable manner. Further in the downward axial direction, a brake pad 104 is arranged that is attached to the brake pad holder 103 and thus, as is the brake pad holder 103, is rotationally coupled to the cage body 44a, but is axially movable with respect to the cage body 44a.

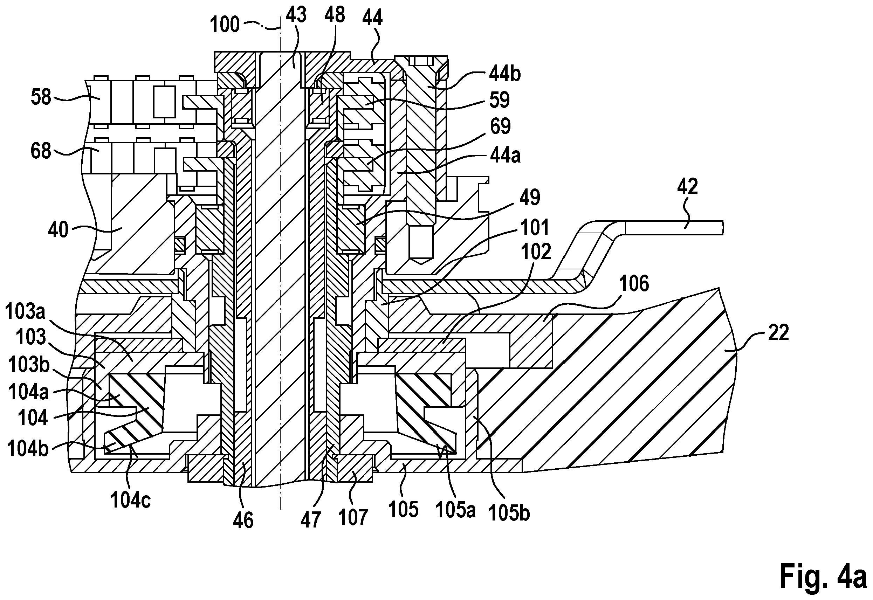

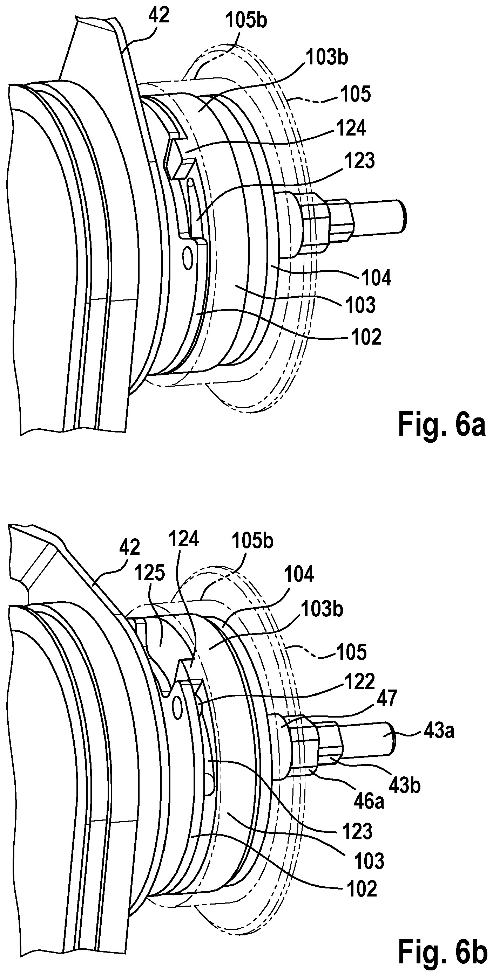

The brake pad holder 103 comprises a disk portion 103a and a cylindrical portion 103b. The disk portion 103a has on its upper side facing the brake toggle plate 102 a multiplicity of sloped notches 123 co-operating with respective cam elements 122 of the brake toggle plate 102 to effect axial movement of the brake pad holder 103 upon turning the brake lever 42, as is described below (see Figs. 6a and 6b). The brake pad 104 is ring-shaped and comprises a base portion 104a and a cone portion 104b. The base portion 104a is encompassed on its outer circumference by the cylindrical portion 103b of the brake pad holder 103. The cone portion 104b forms a substantially conical lip extending in a radially outward direction and, as seen in a radial direction, being inclined downward. On its downward face the cone portion 104b forms a surface that is approximately conical and in the following is denoted contact surface 104c. More exactly speaking, the downward face of the cone portion 104b, i.e. the contact surface 104c, in the uncompressed state as shown in Fig. 3 has a substantially frustoconical shape, having an inner and an outer edge. The outer edge of the cone portion 104b forms a contact edge of the lip. The brake pad 104 is arranged in an approximately concentric manner with respect to the axis 100, and thus an axis of the frustoconical shape approximately coincides with the axis 100. As is explained in detail below, at least the cone portion 104b of the brake pad 104 is elastically deformable (see Figs. 4a-5b).

The brake mechanism relating to the first hand wheel 22 further comprises a hand wheel base plate 105 on which the first hand wheel 22 is fixedly mounted. The hand wheel base plate 105 is supported on the articulation shaft 47, being rotationally coupled to the articulation shaft 47 and thus being rotatable with respect to the support structure, having rotation axis 100. On its side facing the brake pad 104 the hand wheel base plate 105 has an approximately plane surface extending substantially in a radial direction, forming a friction surface 105a co-operating with the contact surface 104c of the lip for frictional coupling. The hand wheel base plate 105 is secured against axial displacement in the downward direction by counter bearing 107. The hand wheel base plate 105, a cylindrical hub element 105b being formed by an axial projection of the hand wheel base plate 105, and a further hub element 106 form a hand wheel core supporting the first hand wheel 22 such that it is rotatable with respect to the axis 100.

The brake mechanism relating to the second hand wheel 23, i.e. to the upper layer 60 and the upper pair of control wires 37, 37', comprises a brake knob 24 fixedly mounted on a brake knob shaft 111 which is pivotably held on a lower section of the axle 43. A brake toggle plate 112 is rigidly connected to the brake knob shaft 111, thus being rotationally coupled to the brake knob 24. Adjacent and parallel to the brake toggle plate 112 a brake pad holder 113 is held on the lower section of the axle 43 in a rotationally fixed, but axially movable manner. Further in the upward axial direction, a brake pad 114 is arranged that is attached to the brake pad holder 113 and thus is non-rotatable, but axially movable with respect to the axle 43.

The brake pad holder 113 comprises a disk portion 113a and a cylindrical portion 113b. The disk portion 113a has on its lower side facing the brake toggle plate 112 a multiplicity of sloped notches 133 co-operating with respective cam elements 132 of the brake toggle plate 112 to effect axial movement of the brake pad holder 113 upon turning the brake knob 24, in a similar manner as described below for the brake toggle mechanism relating to the first hand wheel 22 (see Figs. 6a and 6b).

The brake pad 114 is substantially ring-shaped, being arranged in an approximately concentric manner with respect to the axis 100 and comprising a base portion 114a and a cone portion 114b. The base portion 114a is encompassed on its outer circumference by the cylindrical portion 113b of the brake pad holder 113. In an analogous manner as described above with respect to the brake pad 104, the cone portion 114b is a substantially conical lip, extending in a radially outward direction and being inclined upward. The cone portion 114b on its upper side has an approximately conical or frustoconical contact surface 114c. An axis of the frustoconical shape approximately coincides with the axis 100. In the situation shown in Fig. 3 an inner edge of the frustoconical contact surface 114c is a lower edge and an outer edge, i.e. a contact edge, is an upper edge. Like in the brake pad 104, at least the cone portion 114b of the brake pad 114 is elastically deformable; the brake pad 114 is depicted in Fig. 3 in an uncompressed shape.

The brake mechanism relating to the second hand wheel 23 further comprises a hand wheel base plate 115 to which the second hand wheel 23 is fixed. The hand wheel base plate 115 is fixedly mounted on the articulation shaft 46, thus being rotatable with respect to the support structure, having rotation axis 100 as well. On its side facing the brake pad 114 the hand wheel base plate 115 has an approximately plane face extending substantially in a radial direction, forming a friction surface 115a. The hand wheel base plate 115, a substantially cylindrical hub element 115b, and a further hub element 116 form a hand wheel core of the second hand wheel 23, the second hand wheel 23 being rotatable with respect to the axis 100.

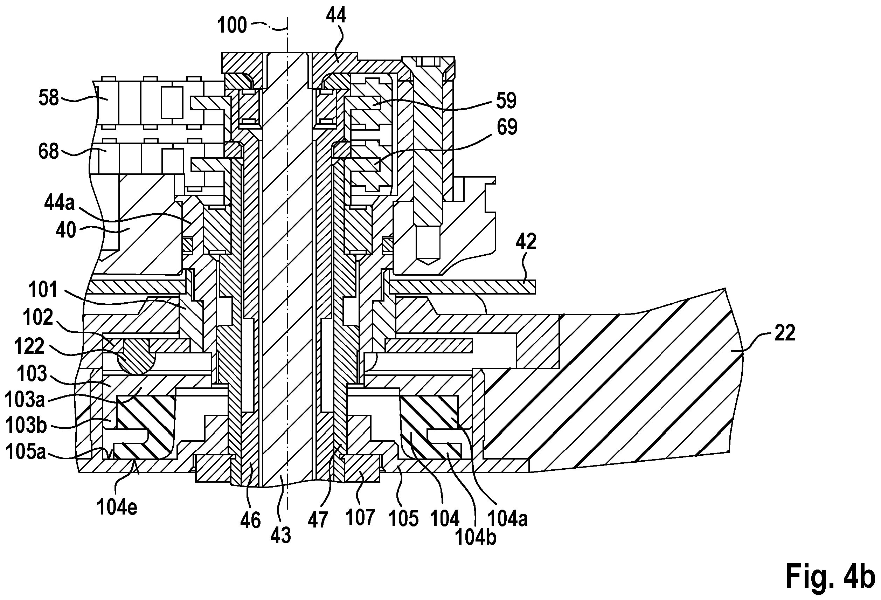

In the following the operation of the brake mechanism of the deflection control mechanism 29 is described with reference to Figs. 4a and 4b showing the brake mechanism of the first hand wheel 22 in an enlarged sectional view wherein the sectional plane is the same as in Fig. 3. Fig. 4a shows the same situation as Fig. 3. As depicted in Fig. 4a, the disk portion 103a of the brake pad holder 103 closely abuts the brake toggle plate 102, i.e. the cam elements 122 of the brake toggle plate 102 are plunged into respective notches 123 (see Figs. 6a and 6b). The brake pad 104 has its original shape, i.e. it is uncompressed, being detached from the friction surface 105a or only touching it lightly. In this state, there is no substantial amount of friction between the brake pad 104 and the hand wheel base plate 105, i.e. the hand wheel 22 can be turned freely to rotate the sprocket 69 of the lower layer 60 via the articulation shaft 47, and the control wires 38 of the lower layer 60 can be moved correspondingly to control deflection of the steerable section 31 of the endoscope shaft 30. In the situation shown in Figs. 3 and 4a the brake mechanism is "off".

On the other hand, in the situation shown in Fig. 4b, the brake lever 42 has been turned. Accordingly, the brake toggle plate 102 has been rotated, such that the cam elements 122 have come out of the respective notches 123. The cam elements 122 touch the disk portion 103a of the brake pad holder 103, pushing the brake pad holder 103 in an axial direction to press the brake pad 104 against the friction surface 105a of the hand wheel base plate 105. As depicted in Fig. 4b, in this state the cone portion 104b of the brake pad 104 contacts the friction surface 105a and is deformed by an axial pressing force. Thereby the cone portion 104b is flattened, such that at least a section of the contact surface 104c assumes a planar radial shape corresponding to the shape of the friction surface 105a of the hand wheel base plate 105. Due to the brake pad 104 frictionally engaging the hand wheel base plate 105, the hand wheel base plate 105 is rotationally coupled to the brake pad holder 103 which in turn is rotationally coupled to the cage body 44a; consequently, rotation of the hand wheel 22 with respect to the support structure is inhibited. In the situation shown in Fig. 4b the brake mechanism of the lower layer 60 is "on".

In Figs. 4a and 4b the extreme positions of the brake pad holder 103 are shown that correspond to the brake-off position and the brake-on position, respectively. In the brake-off position depicted in Fig. 4a, the steerable section 31 of the shaft 30 of the flexible endoscope 10 can be manually controlled by a user in the usual manner by turning the hand wheel 22. On the other hand, by turning the lever 42, the brake mechanism can be switched into the brake-on position shown in Fig. 4b to hold the steerable section 31 of the endoscope shaft 30 in a given deflected state, releasing the user from the need to hold the hand wheels 22.

To resume full operation of the flexible endoscope 10, the user can turn the brake lever 42 back into its previous position, such that the brake toggle plate 102 is rotated back into its position it has in Fig. 4a; thus the brake pad 104 resumes its original shape due to its elastic properties, acting as a spring to push the brake pad holder 103 back into its position it has in Fig. 4a. In this way the brake is released, and deflection of the steerable section 31 can be effected as usual.

Moreover, one or more intermediate states of the brake mechanism may be provided in which the pad 104 is pressed with less force against the friction surface 105a of the hand wheel base plate 105. Such intermediate positions of the brake pad holder 103 correspond to intermediate angular positions of the brake toggle plate 102 and the brake lever 42. The one or more intermediate positions may be marked, for example, haptically or optically to be easily discernible by the user.

The degree of frictional coupling, i.e. a maximal torque at which the hand wheel 22 and the sprocket 69 are held by the brake mechanism depends, in particular, on the force with which the brake pad 104 is pressed against the friction surface 105a, on the friction coefficient between the contact surface 104c and the friction surface 105a, and on the geometry of the cone portion 104b. In order to control the friction coefficient the friction surface 105a may be provided with a lubrication coating introduced for example by applying a solvent in which a small amount of lubricant is dissolved that forms the lubrication coating after evaporation of the solvent. This permits fine adjustment of the maximal torque up to which the hand wheel 22 is blocked.

The action of the brake may be overcome by turning the hand wheel 22 with a torque exceeding the maximal torque that can be transferred by the frictional coupling; further, the frictional coupling may be overcome by an external force acting upon the steerable section 31 of the endoscope shaft 30, exerting a force upon at least one of the pairs of control wires 38 of the lower layer 60 which act to exert a torque on the sprocket 69. Thus, deflection of the steerable section 31 may be enabled even if the brake has not been released.

The second hand wheel 23 relating to the upper layer 50 is equipped with a brake mechanism as shown in Fig. 3 and working in an analogous manner as the brake mechanism relating to the first hand wheel 22 described above. In the situation depicted in Fig. 3, the disk portion 113a of the brake pad holder 113 closely abuts the brake toggle plate 112 the brake pad 114 being substantially detached from the friction surface 115a of the hand wheel base plate 115. In this situation the brake is "off". By turning the knob 24 the brake toggle plate 112 can be turned via the brake knob shaft 111, such that the brake pad holder 113 is pushed by the cam elements 132 in an axial direction, pressing the brake pad 114 against the friction surface 115a of the hand wheel base plate 115. In this way the brake pad 114 can be frictionally coupled to the hand wheel base plate 115, i.e. the brake can be switched "on". Further, one or more intermediate positions having intermediate frictional coupling may be provided.

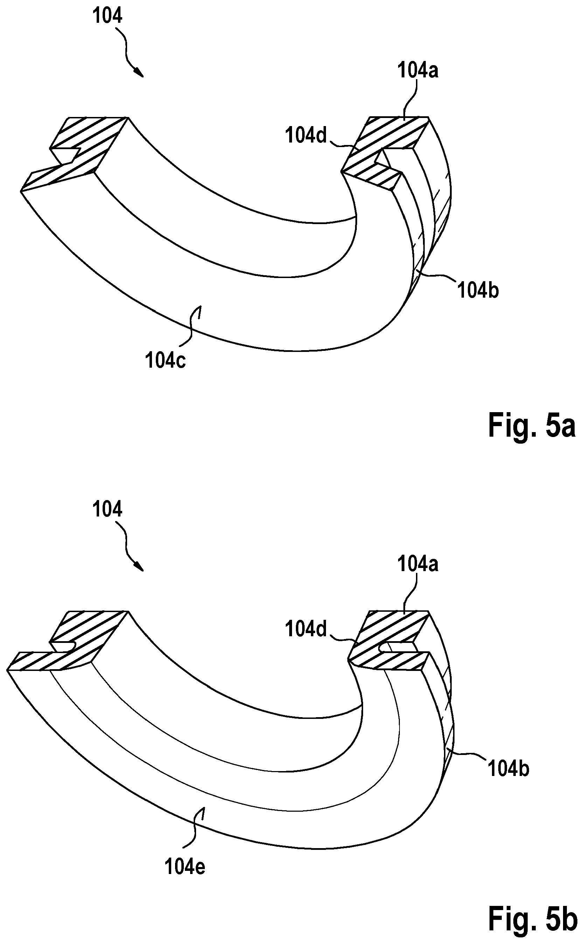

The brake pad 104 of the brake mechanism is depicted in Figs. 5a and 5b in a sectional view in its uncompressed shape, i.e. corresponding to the brake-off state shown in Fig. 4a, and in a compressed or deformed shape it has in the brake-on state shown in Fig. 4b, respectively. The brake pad 104 in total has the shape of an approximately cylindrical ring, a section of about 180° of the ring being shown in Figs. 5a and 5b. The brake pad 104 comprises a base portion 104a which has a substantially rectangular cross-section having a substantially flat radial surface on its upper side. On its lower side at an inner margin the base portion 104a carries a stem portion 104d which in turn carries a cone portion 104b forming an elastically deformable lip. The cone portion 104b has approximately uniform thickness. The lower-side face of the cone portion 104b forms a contact surface 104c for contacting the friction surface 105a.

As can be seen in Fig. 5a, in the uncompressed state of the brake pad 104 the cone portion 104b is inclined with respect to the base portion 104a such that the brake pad 104 at its outer margin has a larger axial extension than at its inner margin. The contact surface 104c of the cone portion 104b has a frustoconical shape. In the example shown, the inclination of the cone portion 104b with respect to a radial direction is about 20°, and the tip of the cone of the frustoconical shape is situated approximately in the center of the upper-side surface of the base portion 104a.

When the brake pad 104 is pressed against the friction surface 105a of the hand wheel base plate 105 at a maximal axial force, part of the contact surface 104c lies against the planar friction surface 105a, forming a substantially planar contact area 104e. As shown in Fig. 5b, an inner section of the cone portion 104b is bent accordingly; additionally or alternatively, the stem section 104d may be bent correspondingly. In this state, the cone portion 104b may protrude in a radial direction beyond the outer circumference of the base portion 104a.

In the example shown the base portion 104a has an approximately rectangular cross-section with dimensions in the radial direction of about 4 mm and about 2-3 mm in the axial direction, having an overall outer diameter of about 25 mm. The stem portion 104d has a radial width of about 2 mm and an axial extension of about 1 mm. The cone portion 104b has a thickness of about 1.5 mm, for example. The inner side of the brake pad 104 is slightly conical. In the axial direction, the brake pad has a total extension of about 6 mm in its uncompressed state and about 5 mm in its fully compressed state, for example; a total range of axial displacement of the brake pad holder 103 relative to the support structure is thus about 1 mm.

The brake pad 104 may be made, for example, of an elastic material, being molded in one piece. The cone portion 104b may be reinforced by steel wires embedded in the molded elastomeric material. In this way the spring rate of the lip can be adapted to a particular application.

The brake toggle mechanism relating to the first hand wheel 22 is further illustrated in Figs. 6a and 6b. The brake toggle plate 102 comprises a cam element 122 formed by a hemispherical nub or a ball-shaped insert. The cam element 122 co-operates with a wedge structure that includes a sloped notch 123 in the upper surface of the disk portion 103a of the brake pad holder 103 such that the brake pad holder 103 is pushed to the right (corresponding to the downward axial direction in Figs. 3, 4a and 4b) by rotation of the brake toggle plate 102. The brake toggle plate 102 comprises a total of three equally spaced cam elements 122, and the disk portion 103a has three corresponding wedge structures to avoid tilting. The hand wheel base plate 105 including the hub element 105b is indicated in Figs. 6a and 6b in a transparent manner.

In the brake-off state shown in Fig. 6a, the cam element 122 is fully entered into the wedge structure of the disk portion 103a, and the brake pad holder 103 closely abuts the toggle plate 102. Consequently, the brake pad 104 is substantially detached from the friction surface of the hand wheel base plate 105. By manually pivoting the lever 42, the brake toggle plate 102 is rotated via rotation of the brake lever shaft 101, such that the cam element 122 is pushed out of the respective notch 123. Thereby the brake pad holder 103 is displaced to the right into such a position that the brake pad 104 is pressed against the friction surface 105a of the hand wheel base plate 105 (Fig. 6b). When the brake lever 42 is turned back into its brake-off position, the brake pad holder 103 is released and is pushed to the left (corresponding to the upward axial direction) by the elastic force of the brake pad 104 (Fig. 6a). Rotation of the brake lever 42 is thus translated into linear axial displacement of the brake pad holder 103. Manufacturing tolerances may be compensated by adjustment shims inserted between the brake pad holder 103 and the brake lever 42 (not shown).

Further, the disk portion 103a of the brake pad holder 103 is provided with a stop element 124 engaging a recess 125 of the brake toggle plate 102 for limiting the pivoting movement of the brake lever 42. Moreover, at both ends of the notch 123 indentations are provided forming detent structures. In the brake-off state shown in Fig. 6a the cam element 122 engages a deep recess provided at one end of the sloped notch 123, while in the brake-on state the cam element 122 engages with a shallow recess formed at the other end of the sloped notch 123. Turning the brake lever 42 out of a respective one of its brake-off and brake-on positions requires application of an increased torque, thus avoiding unintentional activating or releasing the brake.

Moreover, in Figs. 6a and 6b the right-hand ends (corresponding to the downward ends in Fig. 3) of the axle 43 and the articulation shafts 46, 47 are shown. The axle 43 has a cylindrical end section 43a on which the brake knob shaft 111 is rotatably supported, which to the left is followed by a square section 43b having a substantially square cross-section; the brake pad holder 113 has a square cutout to be guided by a corresponding square section 43b of the axle 43 to be axially slidable, but rotationally fixed (see Fig. 3). The articulation shaft 46 has a square end section 46a on which the hand wheel base plate 115 is rigidly held. In this way, rotational coupling and axial movability of the respective elements of the brake mechanism relating to the second hand wheel 23 are accomplished. In an analogous manner the brake pad holder 113 of the brake mechanism relating to the first hand wheel 22 is mounted non-rotatably, but axially slidable on a square section of the cage body 44a, and the hand wheel base plate 105 is rigidly held on the articulation shaft 47 (see Fig. 3).

In the above description the terms "upper", "lower", "upward", and "downward" relate to the orientation of the handpiece 20 and the deflection control mechanism 29 as shown in Figs. 2-4b. Any other orientation may be chosen by a user, according to requirements during use.

For clarity not all reference numerals are displayed in all figures. If a reference numeral is not explicitly mentioned in the description of a figure, it has the same meaning as in the other figures.

### Reference numerals

- 10: Flexible endoscope
- 20: Handpiece
- 21: Housing
- 22: Hand wheel
- 23: Hand wheel
- 24: Knob
- 25: Button
- 26: Connector
- 27: Light cable
- 28: Instrument port
- 29: Deflection control mechanism
- 30: Shaft
- 31: Steerable section
- 32: End cap
- 33: Pivoting element
- 35, 35': Sheath
- 36, 36': Sheath
- 37, 37': Control wire
- 38: Control wire
- 40: Body
- 41: Seal ring
- 42: Lever
- 43: Axle
- 43a: End section
- 43b: Square section
- 44: Cage
- 44a: Cage body
- 44b: Screw
- 46: Articulation shaft
- 46a: End section
- 47: Articulation shaft
- 48: Ball bearing
- 49: Ball bearing
- 50: Upper layer
- 51, 51': Wire coil mounting
- 52, 52': Block
- 56, 56': Block
- 58: Chain
- 59: Sprocket
- 60: Lower layer
- 61, 61': Wire coil mounting
- 62: Block
- 66: Block
- 68: Chain
- 69: Sprocket
- 70: Plate
- 100: Axis
- 101: Brake lever shaft
- 102: Brake toggle plate
- 103: Brake pad holder
- 103a: Disk portion
- 103b: Cylindrical portion
- 104: Brake pad
- 104a: Base portion
- 104b: Cone portion
- 104c: Contact surface
- 104d: Stem portion
- 104e: Contact area
- 105: Hand wheel base plate
- 105a: Friction surface
- 105b: Hub element
- 106: Hub element
- 107: Counter bearing
- 111: Brake knob shaft
- 112: Brake toggle plate
- 113: Brake pad holder
- 113a: Disk portion
- 113b: Cylindrical portion
- 114: Brake pad
- 114a: Base portion
- 114b: Cone portion
- 114c: Contact surface
- 115: Hand wheel base plate
- 115a: Friction surface
- 115b: Hub element
- 116: Hub element
- 120: Cutout
- 121: Cutout
- 122: Cam element
- 123: Notch
- 124: Stop element
- 125: Recess
- 126: Cutout
- 132: Cam element
- 133: Notch

## Claims

1. Deflection control mechanism for a steerable flexible endoscope (10), the endoscope (10) having an elongate flexible shaft (30) comprising a steerable section (31) that is deflectable by movement of at least one elongate control element, the deflection control mechanism (29) comprising a support structure, a drive wheel rotatably supported in or on the support structure, and a hand wheel (22, 23) rotationally coupled to the drive wheel, wherein the deflection control mechanism (29) is connectable to the at least one elongate control element to effect movement of the at least one elongate control element by rotation of the drive wheel, and wherein the deflection control mechanism (29) comprises a brake stator element rotationally coupled to the support structure and a brake rotor element rotationally coupled to the drive wheel, the brake stator element being displaceable relative to the brake rotor element in an axial direction, wherein a first one of the brake stator and brake rotor elements comprises an elastically deformable lip and the other one of the brake stator and brake rotor elements comprises a friction surface (105a, 115a), the elastically deformable lip being arranged for being pressed against the friction surface (105a, 115a) by axial displacement of the brake stator element relative to the brake rotor element, **characterized in that** the elastically deformable lip in an uncompressed state has a substantially frustoconical contact surface (104c, 114c), at least a section of the contact surface (104c, 114c) contacting the friction surface (105a, 115a) in a compressed state.

2. Deflection control mechanism according to claim 1, **characterized in that** the brake stator element comprises the elastically deformable lip and the brake rotor element comprises the friction surface (105a, 115a), and that the brake stator element is displaceable and the brake rotor element is fixed in the axial direction with respect to the support structure.

3. Deflection control mechanism according to claim 1 or 2, **characterized in that** the friction surface (105a, 115a) is a substantially planar radial surface.

4. Deflection control mechanism according to any one of the preceding claims, **characterized in that** the first one of the brake stator and brake rotor elements comprises a brake pad holder (103, 113) to which a brake pad (104, 114) is attached, the brake pad (104, 114) comprising the elastically deformable lip.

5. Deflection control mechanism according to claim 4, **characterized in that** the brake pad (104) comprises a base portion (104a, 114a) carrying the elastically deformable lip, the lip at its free side forming a contact edge or rim for contacting the friction surface (105a, 115a).

6. Deflection control mechanism according to any one of the preceding claims, **characterized in that** the elastically deformable lip is substantially ring-shaped.

7. Deflection control mechanism according to claim 7, **characterized in that** the elastically deformable lip comprises a cone portion (104b, 114b) forming the frustoconical contact surface (104c, 114c) in the uncompressed state, the cone portion (104b, 114b) being deformable by pressing the lip against the friction surface (105a, 115a).

8. Deflection control mechanism according to any one of the preceding claims, **characterized in that** the elastically deformable lip comprises an elastomeric material and/or a composite material.

9. Deflection control mechanism according to any one of the preceding claims, **characterized in that** the friction surface (105a, 115a) exhibits a lubrication coating.

10. Deflection control mechanism according to any one of the preceding claims, **characterized in that** the deflection control mechanism comprises a brake toggle plate (102, 112) co-operating with the first one of the brake stator and brake rotor elements, wherein the first one of the brake stator and brake rotor elements has a wedge structure and the brake toggle plate (102, 112) has a cam element (122, 132) engaging the wedge structure or vice versa to effect axial displacement of the brake stator element relative to the brake rotor element by rotation of the toggle plate (102, 112) with respect to the first one of the brake stator and brake rotor elements, and wherein the wedge structure comprises a detent structure.

11. Deflection control mechanism according to claim 11, **characterized in that** the brake toggle plate (102, 112) has a brake-on rotational position relative to the first one of the brake stator and brake rotor elements in which the lip is pressed at a maximal force to the friction surface (105a, 115a), a brake-off rotational position in which the lip is released from the friction surface (105a, 115a), and a pre-determined intermediate position in which the lip is pressed at an intermediate force against the friction surface (105a, 115a).

12. Deflection control mechanism according to any one of the preceding claims, the deflection control mechanism (29) comprising a further drive wheel rotatably supported in or on the support structure and a further hand wheel (22, 23) rotationally coupled to the further drive wheel, wherein the deflection control mechanism (29) is connectable to at least one further elongate control element to effect movement of the at least one further elongate control element by rotation of the further drive wheel, wherein the deflection control mechanism (29) further comprises a further brake stator element rotationally coupled to the support structure and a further brake rotor element rotationally coupled to the further drive wheel, the further brake stator element being displaceable relative to the further brake rotor element in an axial direction, **characterized in that** a first one of the further brake stator and brake rotor elements comprises a further elastically deformable lip and the other one of the further brake stator and brake rotor elements comprises a further friction surface (105a, 115a), the further elastically deformable lip being arranged for being pressed against the further friction surface (105a, 115a) by axial displacement of the further brake stator element relative to the further brake rotor element.

13. Steerable flexible endoscope having an elongate flexible shaft (30) comprising a steerable section (31) that is deflectable by movement of at least one elongate control element, further comprising a deflection control mechanism (29) in accordance with any one of the preceding claims.

## Patentansprüche

1. Ablenkungssteuerungsmechanismus für ein lenkbares flexibles Endoskop (10), wobei das Endoskop (10) einen länglichen flexiblen Schaft (30) aufweist, der einen lenkbaren Abschnitt (31) umfasst, der durch die Bewegung von mindestens einem länglichen Steuerungselement ablenkbar ist, wobei der Ablenkungssteuerungsmechanismus (29) eine Trägerstruktur, ein Antriebsrad, das drehbar in oder an der Trägerstruktur gelagert ist, und ein Handrad (22, 23) umfasst, das drehbar mit dem Antriebsrad gekoppelt ist, wobei der Ablenkungssteuermechanismus (29) mit dem mindestens einen länglichen Steuerungselement verbindbar ist, um eine Bewegung des mindestens einen länglichen Steuerungselements durch Drehung des Antriebsrads zu bewirken, und wobei der Ablenkungssteuermechanismus (29) ein Bremsstatorelement, das drehbar mit der Trägerstruktur gekoppelt ist, und ein Bremsrotorelement umfasst, das drehbar mit dem Antriebsrad gekoppelt ist, wobei das Bremsstatorelement relativ zu dem Bremsrotorelement in einer axialen Richtung verschiebbar ist, wobei ein erstes der Bremsstator- und Bremsrotorelemente eine elastisch verformbare Lippe umfasst und das andere der Bremsstator- und Bremsrotorelemente eine Reiboberfläche (105a, 115a) umfasst, wobei die elastisch verformbare Lippe angeordnet ist, um durch eine axiale Verschiebung des Bremsstatorelements relativ zu dem Bremsrotorelement gegen die Reiboberfläche (105a, 115a) gedrückt zu werden, **dadurch gekennzeichnet, dass** die elastisch verformbare Lippe in einem nicht zusammengedrückten Zustand eine im Wesentlichen kegelstumpfförmige Kontaktoberfläche (104c, 114c) aufweist, wobei mindestens ein Abschnitt der Kontaktoberfläche (104c, 114c) die Reiboberfläche (105a, 115a) in einem zusammengedrückten Zustand kontaktiert.

2. Ablenkungssteuerungsmechanismus nach Anspruch 1, **dadurch gekennzeichnet, dass** das Bremsstatorelement die elastisch verformbare Lippe umfasst und das Bremsrotorelement die Reiboberfläche (105a, 115a) umfasst, und dass das Bremsstatorelement verschiebbar ist und das Bremsrotorelement in axialer Richtung in Bezug auf die Trägerstruktur feststehend ist.

3. Ablenkungssteuerungsmechanismus nach Anspruch 1 oder 2, **dadurch gekennzeichnet, dass** die Reiboberfläche (105a, 115a) eine im Wesentlichen ebene Radialoberfläche ist.

4. Ablenkungssteuerungsmechanismus nach einem der vorhergehenden Ansprüche, **dadurch gekennzeichnet, dass** das erste der Bremsstator- und Bremsrotorelemente einen Bremsbelaghalter (103, 113) umfasst, an dem ein Bremsbelag (104, 114) angebracht ist, wobei der Bremsbelag (104, 114) die elastisch verformbare Lippe umfasst.

5. Ablenkungssteuerungsmechanismus nach Anspruch 4, **dadurch gekennzeichnet, dass** der Bremsbelag (104) einen Basisabschnitt (104a, 114a) umfasst, der die elastisch verformbare Lippe trägt, wobei die Lippe an ihrer freien Seite eine Kontaktkante oder einen Kontaktrand für den Kontakt mit der Reiboberfläche (105a, 115a) bildet.

6. Ablenkungssteuerungsmechanismus nach einem der vorhergehenden Ansprüche, **dadurch gekennzeichnet, dass** die elastisch verformbare Lippe im Wesentlichen ringförmig ist.

7. Ablenkungssteuerungsmechanismus nach Anspruch 7, **dadurch gekennzeichnet, dass** die elastisch verformbare Lippe einen Kegelabschnitt (104b, 114b) umfasst, der im nicht zusammengedrückten Zustand die kegelstumpfförmige Kontaktoberfläche (104c, 114c) bildet, wobei der Kegelabschnitt (104b, 114b) durch Drücken der Lippe gegen die Reiboberfläche (105a, 115a) verformbar ist.

8. Ablenkungssteuerungsmechanismus nach einem der vorhergehenden Ansprüche, **dadurch gekennzeichnet, dass** die elastisch verformbare Lippe ein elastomeres Material und/oder ein Verbundmaterial umfasst.

9. Ablenkungssteuerungsmechanismus nach einem der vorhergehenden Ansprüche, **dadurch gekennzeichnet, dass** die Reiboberfläche (105a, 115a) eine Schmierbeschichtung aufzeigt.

10. Ablenkungssteuerungsmechanismus nach einem der vorhergehenden Ansprüche, **dadurch gekennzeichnet, dass** der Ablenkungssteuerungsmechanismus eine Bremsumschaltplatte (102, 112) umfasst, die mit dem ersten der Bremsstator- und Bremsrotorelemente zusammenwirkt, wobei das erste der Bremsstator- und Bremsrotorelemente eine Keilstruktur aufweist und die Bremsumschaltplatte (102, 112) ein Nockenelement (122, 132) aufweist, das in die Keilstruktur eingreift oder umgekehrt, um eine axiale Verschiebung des Bremsstatorelements relativ zu dem Bremsrotorelement durch Drehung der Umschaltplatte (102, 112) in Bezug auf das erste der Bremsstator- und Bremsrotorelemente zu bewirken, und wobei die Keilstruktur eine Raststruktur umfasst.

11. Ablenkungssteuerungsmechanismus nach Anspruch 11, **dadurch gekennzeichnet, dass** die Bremsumschaltplatte (102, 112) eine Brems-Ein-Drehposition in Bezug auf das erste der Bremsstator- und Bremsrotorelemente, in der die Lippe mit einer maximalen Kraft an die Reiboberfläche (105a, 115a) gepresst wird, eine Brems-Aus-Drehposition, in der die Lippe von der Reiboberfläche (105a, 115a) gelöst ist, und eine vorbestimmte Zwischenposition aufweist, in der die Lippe mit einer Zwischenkraft gegen die Reiboberfläche (105a, 115a) gepresst wird.

12. Ablenkungssteuerungsmechanismus nach einem der vorhergehenden Ansprüche, wobei der Ablenkungssteuerungsmechanismus (29) ein weiteres Antriebsrad, das drehbar in oder an der Trägerstruktur gelagert ist, und ein weiteres Handrad (22, 23) umfasst, das drehbar mit dem weiteren Antriebsrad gekoppelt ist, wobei der Ablenkungssteuerungsmechanismus (29) mit mindestens einem weiteren länglichen Steuerungselement verbindbar ist, um eine Bewegung des mindestens einen weiteren länglichen Steuerungselements durch Drehung des weiteren Antriebsrads zu bewirken, wobei der Ablenkungssteuerungsmechanismus (29) ferner ein weiteres Bremsstatorelement, das drehbar mit der Trägerstruktur gekoppelt ist, und ein weiteres Bremsrotorelement umfasst, das drehbar mit dem weiteren Antriebsrad gekoppelt ist, wobei das weitere Bremsstatorelement relativ zu dem weiteren Bremsrotorelement in Axialrichtung verschiebbar ist, **dadurch gekennzeichnet, dass** ein erstes der weiteren Bremsstator- und Bremsrotorelemente eine weitere elastisch verformbare Lippe umfasst und das andere der weiteren Bremsstatorund Bremsrotorelemente eine weitere Reiboberfläche (105a, 115a) umfasst, wobei die weitere elastisch verformbare Lippe angeordnet ist, um durch eine axiale Verschiebung des weiteren Bremsstatorelements relativ zu dem weiteren Bremsrotorelement gegen die weitere Reiboberfläche (105a, 115a) gedrückt zu werden.

13. Lenkbares flexibles Endoskop mit einem länglichen flexiblen Schaft (30), umfassend einen lenkbaren Abschnitt (31), der durch die Bewegung von mindestens einem länglichen Steuerungselement ablenkbar ist, ferner umfassend einen Ablenkungssteuerungsmechanismus (29) nach einem der vorhergehenden Ansprüche.

## Revendications

1. Mécanisme de commande de déviation pour un endoscope flexible orientable (10), l'endoscope (10) ayant un arbre flexible allongé (30) comprenant une section orientable (31) qui peut être déviée par le mouvement d'au moins un élément de commande allongé, le mécanisme de commande de déviation (29) comprend une structure de support, une roue d'entraînement supportée de manière rotative dans ou sur la structure de support, et une roue à main (22, 23) couplée en rotation à la roue d'entraînement, dans lequel le mécanisme de commande de déviation (29) peut être relié à l'au moins un élément de commande allongé pour effectuer un mouvement de l'au moins un élément de commande allongé par la rotation de la roue d'entraînement, et dans lequel le mécanisme de commande de déviation (29) comprend un élément de stator de frein couplé en rotation à la structure de support et un élément de rotor de frein couplé en rotation à la roue d'entraînement, l'élément de stator de frein pouvant être déplacé par rapport à l'élément de rotor de frein dans une direction axiale, dans lequel un premier des éléments de stator de frein et de rotor de frein comprend une lèvre élastiquement déformable et l'autre des éléments de stator de frein et de rotor de frein comprend une surface de frottement (105a, 115a), la lèvre élastiquement déformable étant agencée pour être pressée contre la surface de frottement (105a, 115a) par déplacement axial de l'élément de stator de frein par rapport à l'élément de rotor de frein, **caractérisé en ce que** la lèvre élastiquement déformable dans un état non comprimé a une surface de contact sensiblement tronconique (104c, 114c), au moins une section de la surface de contact (104c, 114c) en contact avec la surface de frottement (105a, 115a) dans un état comprimé.

2. Mécanisme de commande de déviation selon la revendication 1, **caractérisé en ce que** l'élément de stator de frein comprend la lèvre élastiquement déformable et l'élément de rotor de frein comprend la surface de frottement (105a, 115a), et que l'élément de stator de frein peut être déplacé et l'élément de rotor de frein est fixe dans la direction axiale par rapport à la structure de support.

3. Mécanisme de commande de déviation selon la revendication 1 ou 2, **caractérisé en ce que** la surface de frottement (105a, 115a) est une surface radiale sensiblement plane.

4. Dispositif de commande de déviation selon l'une quelconque des revendications précédentes, **caractérisé en ce que** le premier des éléments de stator de frein et de rotor de frein comprend un porte-plaquette de frein (103, 113) auquel une plaquette de frein (104, 114) est fixée, la plaquette de frein (104, 114) comprenant la lèvre élastiquement déformable.

5. Mécanisme de commande de déviation selon la revendication 4, **caractérisé en ce que** la plaquette de frein (104) comprend une partie base (104a, 114a) portant la lèvre élastiquement déformable, la lèvre, au niveau de son côté libre, formant un bord de contact ou un rebord pour entrer en contact avec la surface de frottement (105a, 115a).

6. Dispositif de commande de déviation selon l'une quelconque des revendications précédentes, **caractérisé en ce que** la lèvre élastiquement déformable est sensiblement en forme d'anneau.

7. Mécanisme de commande de déviation selon la revendication 7, **caractérisé en ce que** la lèvre élastiquement déformable comprend une partie cône (104b, 114b) formant la surface de contact tronconique (104c, 114c) dans l'état non comprimé, la partie cône (104b, 114b) étant déformable par pression de la lèvre contre la surface de frottement (105a, 115a).

8. Dispositif de commande de déviation selon l'une quelconque des revendications précédentes, **caractérisé en ce que** la lèvre élastiquement déformable comprend un matériau élastomère et/ou un matériau composite.

9. Dispositif de commande de déviation selon l'une quelconque des revendications précédentes, **caractérisé en ce que** la surface de frottement (105a, 115a) présente un revêtement de lubrification.

10. Dispositif de commande de déviation selon l'une quelconque des revendications précédentes, **caractérisé en ce que** le mécanisme de commande de déviation comprend une plaque de basculement de frein (102, 112) coopérant avec le premier des éléments de stator de frein et de rotor de frein, dans lequel le premier des éléments de stator de frein et de rotor de frein a une structure en coin et la plaque de basculement de frein (102, 112) a un élément de came (122, 132) venant en prise avec la structure en coin ou inversement pour effectuer un déplacement axial de l'élément de stator de frein par rapport à l'élément de rotor de frein par rotation de la plaque de basculement (102, 112) par rapport au premier des éléments de stator de frein et de rotor de frein, et dans lequel la structure en coin comprend une structure de cran.

11. Mécanisme de commande de déviation selon la revendication 11, **caractérisé en ce que** la plaque de basculement de frein (102, 112) a une position de rotation d'application de frein par rapport au premier des éléments de stator de frein et de rotor du frein dans laquelle la lèvre est pressée avec une force maximale sur la surface de frottement (105a, 115a), une position de rotation de non application de frein dans laquelle la lèvre est libérée de la surface de frottement (105a, 115a), et une position intermédiaire prédéterminée dans laquelle la lèvre est pressée avec une force intermédiaire contre la surface de frottement (105a, 115a).

12. Dispositif de commande de déviation selon l'une quelconque des revendications précédentes, le mécanisme de commande de déviation (29) comprend une autre roue d'entraînement supportée de manière rotative dans ou sur la structure de support et une autre roue à main (22, 23) couplée en rotation à l'autre roue d'entraînement, dans lequel le mécanisme de commande de déviation (29) peut être relié à au moins un autre élément de commande allongé pour effectuer un mouvement de l'au moins un autre élément de commande allongé par rotation de l'autre roue d'entraînement, dans lequel le mécanisme de commande de déviation (29) comprend en outre un autre élément de stator de frein couplé en rotation à la structure de support et un autre élément de rotor de frein couplé en rotation à l'autre roue d'entraînement, l'autre élément de stator de frein pouvant être déplacé par rapport à l'autre élément de rotor de frein dans une direction axiale, **caractérisé en ce qu'un** premier des autres éléments de stator de frein et de rotor de frein comprend une autre lèvre élastiquement déformable et l'autre des autres éléments de stator de frein et de rotor de frein comprend une autre surface de frottement (105a, 115a), l'autre lèvre élastiquement déformable étant agencée pour être pressée contre l'autre surface de frottement (105a, 115a) par déplacement axial de l'autre élément de stator de frein par rapport à l'autre élément de rotor de frein.

13. Endoscope flexible orientable ayant un arbre flexible allongé (30) comprenant une section orientable (31) qui peut être déviée par un mouvement d'au moins un élément de commande allongé, comprenant en outre un mécanisme de commande de déviation (29) conformément à l'une quelconque des revendications précédentes.
